# DEMANDE DE BREVET EUROPEEN

(11) **EP 0 664 996 A1**
(43) Date de publication de la demande: **02.08.1995**
(21) Numéro de dépôt: 95400065.9
(22) Date de dépôt: 13.01.1995
(51) Int. Cl.: A61F 7/00, G10K 11/34

(54) **Procédé de commande d'un appareil de traitement par hyperthermie à l'aide d'ultrasons**

(30) Priorité: 27.01.1994 FR 9400904
(71) Demandeur: EDAP INTERNATIONAL, F-77200 Croissy Beaubourg-Marne la Vallée (FR)
(72) Inventeur: Dory, Jacques, F-77580 Villiers-Sur-Morin (FR)
(74) Mandataire: Hirsch, Marc-Roger

(57) **Abrégé**

Le procédé de commande d'un appareil de traitement par hyperthermie à l'aide d'ultrasons consiste à émettre des ultrasons focalisés sur une tache focale, et à faire varier la position de ladite tache focale par rapport au transducteur, et est caractérisé en ce la position de ladite tache focale par rapport audit transducteur varie grâce aux seules variations des phases relatives des signaux électriques excitant les éléments piézo-électriques, et en ce que la puissance de crête de la tache focale et le recouvrement entre les différentes positions de la tache focale sont tels que l'énergie rayonnée au cours d'un tir en tout point de la tache focale équivalente est sensiblement égale à l'énergie nécessaire pour détruire les cellules.

L'invention a aussi pour objet un appareil de traitement par hyperthermie pour la mise en oeuvre du procédé.

## Description

La présente invention a pour objet un procédé de commande d'un appareil de traitement par hyperthermie à l'aide d'ultrasons comprenant un transducteur avec une pluralité d'éléments piézo-électriques excités chacun par un signal électrique, ledit procédé consistant à émettre des ultrasons focalisés sur une tache focale, et à faire varier la position de ladite tache focale par rapport audit transducteur, la position de la tache focale variant par rapport au transducteur à une vitesse supérieure à la vitesse de diffusion thermique dans la zone à traiter.

L'invention a aussi pour objet un appareil de traitement par hyperthermie, comprenant un transducteur avec une pluralité d'éléments piézo-électriques excités par une pluralité de signaux électriques d'excitation, et des moyens de déphasage des dits signaux électriques d'excitation.

Les ondes ultrasonores, appelées couramment ultrasons, peuvent se propager dans le corps humain. Ils peuvent également être facilement focalisés. Ces propriétés sont utilisées en médecine pour agir à distance sur les structures internes, de façon non invasive et sans traitement chirurgical. Dans ce but, des ondes ultrasonores sont focalisées dans une zone appelée foyer ou tache focale. Pour le traitement, le foyer est amené sur la zone à traiter.

En traitement par hyperthermie, la focalisation des ultrasons provoque au foyer un échauffement des tissus qui conduit à leur destruction.

Des appareils connus de traitement par hyperthermie sont déjà décrits, par exemple dans les documents EP-A-0 162 735 (coupelle sphérique et tache focale fixe, de diamètre de l'ordre de 2 ou 3 mm), EP-A-0 370 841 (transducteur mobile mécaniquement), ou EP-A-0 194 897 (balayage de la zone à traiter par déplacement mécanique du transducteur) . Une description plus complète de ces divers dispositifs et de leurs inconvénients est donnée dans la demande de brevet français 9400904 (priorité de la présente demande), dont le contenu est incorporé par référence.

Dans le document Ebbeni et al, *A spherical section ultrasound phased array for deep localisation hyperthermia,* IEEE Transactions on Biomedical Engineering, vol. 38 no 7, 01/07/91, pages 634-643 sont décrits les résultats d'une simulation informatique d'émission d'ultrasons par une coupelle sphérique présentant une pluralité d'éléments piézo-électriques. Il est suggéré de déplacer la tache focale par variation des amplitudes et des phases des différents éléments piézo-électriques, de façon à irradier une pluralité de points de contrôle disposés sur un cercle, ou sur deux cercles concentriques. Il est toutefois considéré comme préférable dans ce document de générer directement un champ ultrasonore présentant la dite pluralité de points de contrôle.

Le document US-A-4 938 217 décrit un dispositif d'hyperthermie, comprenant un transducteur présentant une pluralité d'éléments piézo-électriques, combinant déplacement mécanique du transducteur et modification de la longueur de la tache focale ou de sa distance par rapport au transducteur par déphasage des signaux d'excitation et apodisation. Dans ce document, la variation de longueur de la tache focale au cours de la rotation du transducteur permet de balayer des zones de forme irrégulière.

Ces divers dispositifs connus présentent des inconvénients.

Il est difficile de synthétiser des taches focales de grande dimension, comme dans le document EP-A-0 194 897, ou présentant une pluralité de pics, comme dans le document Ebbeni et al: d'une part, ceci implique des calculs complexes de phase et d'amplitude des signaux d'excitation. D'autre part, dans le cas de dispositifs réels présentant un nombre raisonnable d'éléments piézo-électriques, ceci engendre autour de la tache focale des irrégularités qui peuvent conduire à des lésions.

Si au contraire, on utilise des taches focales de dimensions réduites, il devient difficile de traiter des volumes importants. Un balayage mécanique de la zone à traiter, en déplaçant le transducteur (EP-A-0 194 897 ou US-A-4 938 217), implique de disposer de moyens mécaniques pour déplacer le transducteur, ce qui conduit à des appareillages encombrants et compliqués. Par ailleurs, de nombreux tirs sont nécessaires, et si la zone à traiter bouge avec la respiration du patient, les points d'impacts se répartissent de façon aléatoire à l'intérieur du volume théoriquement balayé. La zone à traiter peut ne pas être complètement balayée (lacunes), et des points d'impact peuvent même se situer en dehors de cette zone.

Le déplacement électronique de la tache focale avec une apodisation, telle que suggérée dans le document Ebbeni et al ou dans le document US-A-4 938 217 est une technique complexe à mettre en oeuvre; il est difficile de modifier la puissance de crête des ondes ultrasonores émises par chacun des éléments piézo-électriques, surtout dans le cadre de modifications rapides.

Un problème supplémentaire est la répartition de l'énergie dans la tache focale. Dans le document Ebbeni et al, il est proposé pour déterminer l'énergie nécessaire pour "atteindre et maintenir un certain niveau d'hyperthermie", de calculer une moyenne spatio-temporelle dans le volume de la tumeur de l'énergie déposée au niveau des points de contrôle. Ce genre d'approche ne peut fonctionner que dans le cas d'une hyperthermie dite "classique", i.e. pour des volumes importants et des températures modérées (43 à 45°C).

Dans le document US-A-4 938 217, il est simplement mentionné qu'il est souhaitable d'obtenir une température uniforme dans la tumeur, et d'éviter un échauffement excessif. Rien dans ce document n'indique la façon dont on peut choisir l'énergie au niveau de la tache focale ou des différents points de la zone à traiter.

Dans les dispositifs connus, les pertes par diffusion représentent une part importante de l'énergie rayonnée dans le patient. Une grande partie de l'énergie rayonnée n'est pas utilisée pour la destruction des cellules. Ceci pose un problème dans la mesure où l'excès d'énergie rayonnée peut conduire à des lésions par brûlure, soit des tissus intérieurs, soit de la peau.

La présente invention propose une solution à ces différents inconvénients. Elle résout le problème de la destruction par hyperthermie d'une zone à traiter, avec une déperdition d'énergie minimale, quelle que soit la forme de la zone à traiter. Elle s'applique tout particulièrement à la pyrothérapie, i.e. à l'hyperthermie à des températures élevées (par exemple supérieures à 48°C), qui permettent une destruction quasi instantanée des cellules.

L'invention propose un procédé de commande d'un appareil de traitement par hyperthermie à l'aide d'ultrasons, comprenant un transducteur avec une pluralité d'éléments piézo-électriques excités chacun par un signal électrique, ledit procédé consistant à émettre des ultrasons focalisés sur une tache focale, et à faire varier la position de ladite tache focale par rapport audit transducteur ou par rapport au support du dit transducteur, la position de la tache focale variant par rapport au transducteur ou par rapport au support du dit transducteur à une vitesse supérieure à la vitesse de diffusion thermique dans la zone à traiter, caractérisé en ce que la puissance de crête de la tache focale et le recouvrement entre les différentes positions de la tache focale sont tels que l'énergie rayonnée au cours d'un tir en tout point de la tache focale équivalente est sensiblement égale à l'énergie nécessaire pour détruire les cellules.

Grâce à ce procédé, la quantité d'énergie rayonnée au cours du traitement est minimale; on évite ainsi le risque de brûlure en dehors de la zone traitée. De plus, la destruction des tissus est assurée.

Dans un mode de réalisation de ce procédé, la position de ladite tache focale par rapport audit transducteur varie grâce aux seules variations des phases relatives des signaux électriques excitant lesdits éléments piézo-électriques.

Dans un mode de mise en oeuvre de ce procédé, la dite tache focale équivalente est balayée au cours d'un tir de façon homogène.

Ceci est particulièrement avantageux lors d'un premier tir.

Selon une variante de l'invention, ladite tache focale équivalente est balayée au cours d'un tir de façon non homogène, de sorte à compléter l'énergie provenant du ou des tirs précédents.

Ceci permet de tenir compte de la diffusion thermique ou du dépôt d'énergie dans les couches voisines.

Dans une variante de l'invention, les ultrasons sont émis au cours d'un tir sous forme de trains d'ondes séparés par des blancs, et la position de ladite tache focale varie par rapport au transducteur pendant lesdits blancs.

Dans une autre variante de l'invention, les ultrasons sont émis au cours d'un tir sous forme de trains d'ondes continus, et la position de ladite tache focale varie par rapport au transducteur sans interruption de l'émission des ultrasons.

Ces deux variantes sont aussi simples à mettre en oeuvre.

L'invention propose que ladite tache focale présente une forme allongée, et en ce qu'elle se déplace par rapport au transducteur sensiblement selon sa direction longitudinale.

L'invention propose en outre un appareil de traitement par hyperthermie, comprenant un transducteur avec une pluralité d'éléments piézo-électriques excités par une pluralité de signaux électriques d'excitation, et des moyens de déphasage des dits signaux électriques d'excitation, caractérisé en ce qu'il comprend une pluralité d'émetteurs émettant chacun un signal électrique d'excitation vers un des dits éléments piézo-électriques, un pilote haute fréquence émettant un signal pilote commun vers les dits émetteurs, et un générateur d'adresses, et en ce que les moyens de déphasage comprennent dans chacun des dits émetteurs des moyens pour déphaser le dit signal pilote en fonction de l'adresse reçue depuis le dit générateur d'adresses.

Dans un mode de réalisation, cet appareil de traitement par hyperthermie est tel que, dans chacun des dits émetteurs les moyens pour déphaser le dit signal pilote comprennent un registre à décalage et des moyens de mémoire commandant le dit registre à décalage en fonction de l'adresse reçue depuis le dit générateur d'adresses, et que les dits moyens de mémoire sont préprogrammés en fonction de la position de l'émetteur dans lequel ils se trouvent.

Dans ce cas, chacun des dits registres à décalage peut être susceptible d'induire sur le signal pilote des déphasages variant de seizième de période en seizième de période. Dans un autre mode de réalisation, chacun des dits registres à décalage peut être susceptible d'induire sur le signal pilote des déphasages variant de huitième de période en huitième de période.

Les avantages et caractéristiques de la présente invention ressortiront mieux de la description suivante, donnée uniquement à titre d'exemple, et où:
- la figure 1 montre l'allure du profil de l'intensité au niveau de la tache focale dans un dispositif selon l'art antérieur;
- la figure 2 montre un schéma d'un appareil pour la mise en oeuvre du procédé selon l'invention;
- la figure 3 montre un mode de réalisation de l'émetteur d'un appareil de la figure 1;
- la figure 4a montre l'allure du profil de l'intensité au niveau de la tache focale dans un dispositif selon l'invention;
- la figure 4b montre l'allure du profil de l'intensité au niveau de la tache focale équivalente dans un dispositif selon l'invention;
- la figure 5 montre l'évolution de la température au cours du temps, au niveau d'une tache focale synthétique selon l'invention;
- la figure 6 montre une répartition d'intensité sur la tache focale équivalente, permettant de maintenir une température constante;
- la figure 7 montre une répartition d'intensité dans le plan de la tache focale équivalente, et dans un plan situé en avant;
- la figure 8 montre un schéma d'un autre mode de réalisation du dispositif de la figure 1;
- la figure 9 montre un chronogramme des signaux dans le circuit de la figure 8;
- la figure 10 montre un mode de réalisation d'un appareil pour la mise en oeuvre de l'invention;
- la figure 11 montre un schéma plus précis d'un mode de réalisation du circuit de validation de la figure 10;
- la figure 12 montre une forme possible de tache focale et de tache focale équivalente selon l'invention.

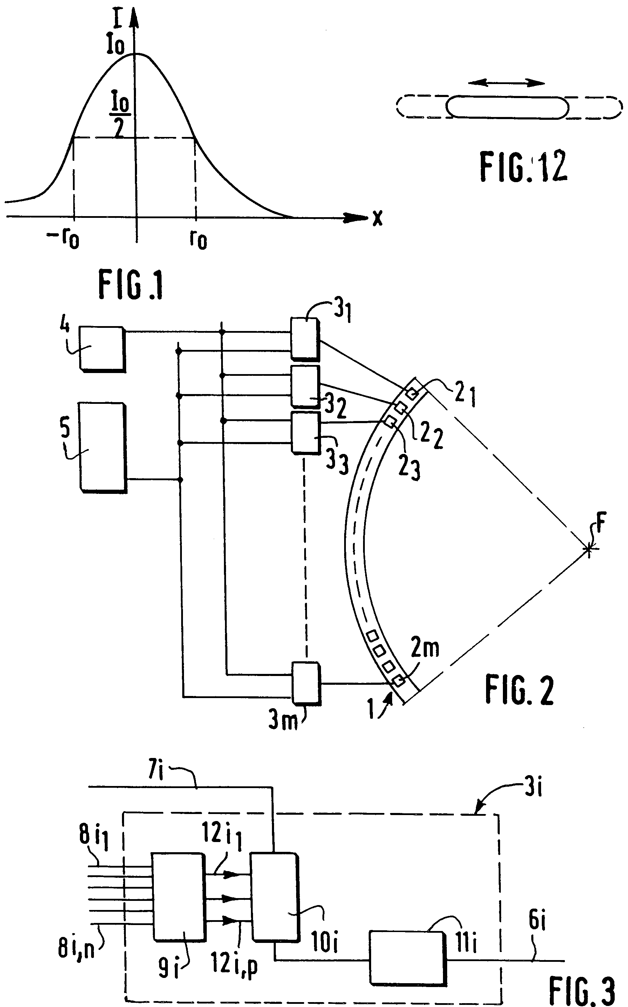

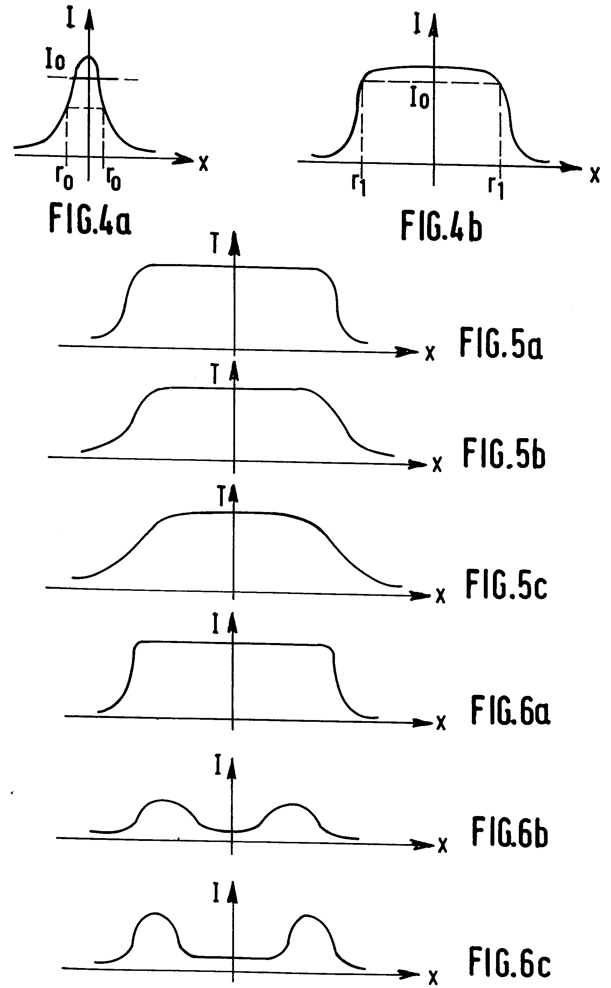

La figure 1 montre l'allure du profil de l'intensité au niveau du foyer dans un dispositif selon l'art antérieur, tel que celui décrit dans le document EP-A-0 370 841. Cet appareil comprend un transducteur de puissance en forme de coupelle sphérique, qui concentre les ultrasons en un foyer situé au centre géométrique de la coupelle. Du fait de la forme de la coupelle, le foyer présente une symétrie de révolution autour de l'axe de symétrie de la coupelle. La figure 1 montre l'intensité au niveau du foyer, le long d'une droite passant par le foyer et perpendiculaire à l'axe de symétrie de la coupelle. En abscisse est portée la distance à l'axe de symétrie de la coupelle et en ordonnée l'intensité du rayonnement ultrasonore.

La courbe de la figure 1 présente une allure de cloche, l'intensité ultrasonore diminuant progressivement du centre de la tache focale vers sa périphérie. On considère généralement que le diamètre de la tache focale correspond à un cercle de rayon rₒ, centré sur l'axe de symétrie du faisceau ultrasonore, et à l'intérieur duquel l'intensité ultrasonore est supérieure à la moitié de l'intensité Iₒ sur l'axe du faisceau (diamètre à 6 dB).

Compte tenu des problèmes de mouvements de la zone à traiter, on choisit dans les dispositifs de l'art antérieur l'intensité du faisceau ultrasonore et la durée d'un tir de sorte que les zones à traiter se trouvant à l'intérieur de la tache focale soient complètement détruites en un seul tir. On choisit donc l'intensité du faisceau de sorte qu'au bord de la tache focale, l'intensité Iₒ/2 assure la destruction des tissus à traiter en un seul tir.

A l'intérieur de la tâche focale, l'intensité ultrasonore est supérieure à Iₒ/2, et la partie supérieure à Iₒ/2 correspond à une énergie inutile. De même, à l'extérieur de la tache focale, l'énergie est non nulle, et insuffisante pour détruire les cellules. Ceci correspond aussi à une énergie inutile. Cette énergie excédentaire augmente le risque de brûlure superficielle du patient. De plus, elle augmente la température des tissus autour de la tache focale. Si la cadence de tir est trop élevée, ceci peut conduire à des lésions en dehors de la zone à traiter.

Dans les systèmes de l'art antérieur présentant une telle répartition de l'énergie rayonnée dans le tissus à traiter, l'énergie totale rayonnée est de l'ordre de 2,5 fois l'énergie théoriquement nécessaire pour détruire les tissus à l'intérieur de la tache focale.

A titre d'exemple, dans un dispositif tel que celui décrit dans EP-A-0 370 841, la tache focale présente un diamètre de l'ordre de 2 mm. Au bout d'un temps appelé temps de stabilisation, la température dans la tache focale cesse de monter, car les pertes par diffusion compensent exactement les apports d'énergie ultrasonore. Dans le dispositif connu, ce temps de stabilisation est de l'ordre de 2 secondes, pour une puissance acoustique de l'ordre de 10 kW. Pour une puissance acoustique d'environ 10 kW, les tissus se trouvant au niveau de la tache focale sont détruits par l'onde ultrasonore après un tir unique durant environ 0,1 s. Ce temps est inférieur au temps de stabilisation.

La figure 2 montre un schéma d'un appareil pour la mise en oeuvre du procédé selon l'invention. Comme les dispositifs connus, l'appareil de la figure 2 comporte un transducteur 1 comprenant une pluralité m d'éléments piézo-électriques 2₁, 2₂, ..., 2ₘ chacun excité par un signal électrique. Dans le schéma de la figure 2, le transducteur 1 présente la forme d'une coupelle sphérique, mais l'invention n'est pas limitée à cette forme. Le transducteur pourrait aussi être constitué de plusieurs éléments présentant chacun une pluralité d'éléments piézo-électriques.

Avantageusement, le transducteur est couplé à un dispositif d'imagerie, par exemple par échographie, comme décrit dans le document EP-A-0 162 735. Le transducteur 1 comprend aussi un dispositif non représenté permettant le couplage ultrasonore, par exemple constitué d'une cavité solidaire des transducteur 1, fermée par une membrane souple et remplie de liquide.

Les ondes ultrasonores émises par les éléments piézo-électriques 2₁ à 2ₘ sont concentrées en une tache focale. Dans le cas d'un transducteur présentant la forme d'une coupelle sphérique, la tache focale est située au niveau du centre géométrique F de la coupelle, lorsque les signaux d'excitation des éléments piézo-électriques ont tous la même phase.

Chaque élément piézo-électrique 2₁...2ₘ du transducteur 1 est excité par un signal électrique à haute fréquence provenant d'un émetteur correspondant 3₁...3ₘ. Les émetteurs 3₁...3ₘ sont reliés d'une part à un pilote haute fréquence 4 et d'autre part à un générateur d'adresse 5. Le pilote haute-fréquence 4 émet un signal pilote haute fréquence qui constitue une source commune à tous les émetteurs et dont le fonctionnement est décrit plus en détail ci-dessous. Le fonctionnement du générateur d'adresses sera mieux compris en référence à la description de la figure 3.

L'appareil de la figure 2 comprend en outre des moyens de commande non représentés, commandant le fonctionnement du pilote haute fréquence 4 et du générateur d'adresse 5. Ces moyens de commande sont susceptibles d'être programmés par un opérateur.

La figure 3 montre un schéma d'un mode de réalisation d'un émetteur 3ᵢ de l'appareil de la figure 2. L'émetteur 3ᵢ est destiné à émettre vers un élément piézo-électrique 2ᵢ un signal électrique d'excitation, par l'intermédiaire d'un conducteur 6ᵢ. L'émetteur 3ᵢ reçoit du pilote haute fréquence 4 un signal pilote, par un conducteur 7ᵢ. Il reçoit aussi du générateur d'adresse 5 un signal d'adresse, sur une pluralité n de conducteurs 8_{i,1} à 8_{i,n}.

L'émetteur 3ᵢ comprend des moyens de mémoire, par exemple une mémoire morte type ROM 9ᵢ (ou tout autre type de mémoire), un registre à décalage 10ᵢ, et un dispositif d'émission 11ᵢ. La mémoire morte 9ᵢ reçoit les signaux en provenance du générateur d'adresse 5, par les conducteurs 8_{i,1} à 8_{i,n}; elle émet un signal de commande vers le registre à décalage 10ᵢ, par p conducteurs 12_{i,1} à 12_{i,p}. Le registre à décalage 10ᵢ reçoit par le conducteur 7ᵢ, le signal pilote provenant du pilote 4 représenté à la figure 2. Il émet un signal vers le dispositif d'émission 11ᵢ par un conducteur 13ᵢ. Le dispositif d'émission 11ᵢ reçoit par le conducteur 13ᵢ le signal provenant du registre à décalage 10ᵢ et émet par le conducteur 6ᵢ un signal électrique d'excitation vers l'élément piézo-électrique 2ᵢ.

La réalisation de l'appareil décrit ci-dessus est à la portée de l'homme du métier, à partir de composants classiques.

Selon l'invention, la position de la tache focale du transducteur 1 varie au cours du traitement. La variation de la position de la tache focale est obtenue par variation des phases relatives des signaux électriques excitant lesdits éléments piézo-électriques, comme expliqué maintenant.

Le pilote haute-fréquence 4 émet un signal pilote haute fréquence vers l'ensemble des émetteurs 3₁ à 3ₘ. Ce signal est reçu dans chaque émetteur 3i par le registre à décalage 10ᵢ. Le signal reçu subit une variation de phase δφᵢ dans le registre à décalage. Le signal ainsi déphasé sert de commande pour l'émission par le dispositif d'émission 11ᵢ vers l'élément piézo-électrique 2ᵢ.

Par ailleurs, le générateur d'adresse 5, pour une position donnée de la tache focale, génère sur le conducteur 8_{1,1} à 8_{m,n} des signaux d'adresses correspondant à la position souhaitée de la tache focale. Ces signaux d'adresses sont reçus dans chacun des émetteurs 3ᵢ par la mémoire morte 9ᵢ La mémoire morte 9ᵢ en réponse aux signaux d'adresse, émet des signaux de commande vers le système de décalage 10ᵢ.

La mémoire morte 9ᵢ de chaque émetteur 3ᵢ est programmée à l'avance, en fonction de la position de l'élément piézo-électrique 2ᵢ correspondant, pour émettre vers le registre à décaler 10ᵢ des signaux de commande induisant dans le registre à décalage une variation de phase δφᵢ. Cette variation de phase est celle nécessaire sur l'élément piézo-électrique 2ᵢ pour positionner la tâche focale à la position souhaitée, telle que donnée par les signaux d'adresse.

Dans un mode de réalisation de l'invention, on programme l'adresse mémoire dans le générateur d'adresse sur n = 13 bits. On obtient ainsi un balayage possible sur 2ⁿ = 8192 positions de la tache focale, soit 1024 dans un plan perpendiculaire à l'axe de la cible, et sur 8 profondeurs différentes.

Dans ce cas, il suffit pour les registres à décalage 10ᵢ d'une précision d'un seizième de période: de ce fait, on peut choisir une valeur de 4 pour l'entier p.

Dans le cadre d'un transducteur en forme de coupelle, d'un diamètre de 300 mm environ, présentant une distance focale de l'ordre de 320 mm et 160 éléments piézo-électriques de 20 mm de diamètre, la tache focale présente un diamètre à 6 dB de l'ordre de 2 mm. Le choix de p = 4 pour les registres à décalage permet dans un plan perpendiculaire à l'axe de symétrie de la coupelle, d'obtenir une variation de position de la tache focale avec un pas de l'ordre de 0,5 mm.

Selon l'invention, il est aussi possible d'utiliser une résolution sur le déphasage d'un huitième de période. Dans ce cas, les phases des signaux d'excitation des éléments piézo-électriques varient de huitième de période en huitième de période. On choisit alors une valeur de 3 pour l'entier p.

L'appareil décrit en référence aux figures 2 et 3 permet ainsi de faire varier la position de la tache focale par rapport au transducteur 1. Selon l'invention, on fait varier cette position de façon à constituer au cours d'un tir une zone appelée tache focale équivalente.

La figure 4a montre l'allure du profil d'intensité au niveau de la tache focale d'un transducteur du type de celui décrit ci-dessus. La courbe de la figure 4a est la même que celle de la figure 1, à une échelle différente sur l'axe des abscisses.

La figure 4b montre l'allure du profil d'intensité au niveau de la tache focale équivalente obtenue selon l'invention.

On décrit d'abord un exemple de mise en oeuvre de l'invention; dans cet exemple, on utilise un transducteur présentant une tache focale de rayon rₒ à 6 dB de l'ordre de 1 mm. Au cours d'un tir, on fait varier la position de la tache focale par rapport au transducteur, en déplaçant la tache focale d'une distance de l'ordre de son rayon rₒ, avec une périodicité de l'ordre de 1 ms.

Pour cela, le pilote haute fréquence 4 émet un signal périodique, constitué de trains d'ondes d'une fréquence de l'ordre de 1 MHz, d'une durée d'une milliseconde, séparés par des blancs d'une durée de 5 microsecondes. Pendant chaque blanc, on fait varier la position de la tache focale, à l'aide du générateur d'adresses 5. Comme l'amplitude des signaux d'excitation émis par le pilote haute fréquence 4 est toujours identique, et que l'on n'utilise pas d'apodisation, cette durée de 5 µs est suffisante pour que les moyens de mémoire 9ᵢ reçoivent les signaux provenant du générateur d'adresse et programment en conséquence les registres à décalage 10ᵢ.

On programme les moyens de commande de l'appareil de façon à balayer une tache focale équivalente d'une surface environ 50 fois supérieure à la surface de la tache focale, et présentant une forme de disque. Cette tache focale équivalente est balayée en une durée de l'ordre de 0.1 s, en faisant varier 100 fois la position de la tache focale; dans le cas du balayage d'une tache focale équivalente en forme de disque, la surface balayée présente un diamètre de l'ordre de 15 mm (√50 x 2 mm); le recouvrement entre les différentes positions voisines de la tache focale est de l'ordre de 50%. La diffusion thermique pendant cette durée est négligeable, et l'allure du profil d'intensité dans la tache focale équivalente, au bout de cette durée de 0.1 s est représentée à la figure 4b, à la même échelle que la figure 4a.

Comme on le voit à la figure 4b, du fait de la variation de position de la tache focale au cours du temps, la répartition d'intensité dans la tache focale équivalente est proche d'une répartition d'énergie optimale, en forme de créneau.

De la sorte, en notant Iₒ l'intensité nécessaire pour détruire les cellules en une durée de 0,1 s, on peut choisir pour la tache focale une intensité de crête de l'ordre de 1,2.Iₒ. Dans ce cas, avec un balayage tel que celui décrit ci-dessus, on assure la destruction de toutes les cellules de la cible.

L'énergie totale rayonnée pour détruire les tissus avec un tel profil de répartition d'intensité est de l'ordre de 1.2 fois l'énergie théoriquement nécessaire. L'invention permet ainsi de limiter la quantité d'énergie rayonnée.

De la sorte, on obtient une tache focale équivalente présentant un diamètre important, sans aberrations environnantes du champ ultrasonore. La répartition de l'intensité ultrasonore dans la tache focale équivalente est telle que l'énergie rayonnée est quasiment complètement utilisée pour la destruction, en limitant les pertes par diffusion. L'invention permet ainsi de pallier aux inconvénients de l'art antérieur.

Dans un autre exemple de mise en oeuvre de l'invention, le pilote haute fréquence n'émet pas un signal dont l'enveloppe présente une forme de créneau, mais émet pendant le tir un signal coninu. La position de la tache focale varie sans interruption de l'émission ultrasonore, avec une vitesse ou un pas de déplacement tel que l'énergie excédentaire du profil d'intensité de la figure 4a soit utilisée dans les positions voisines. On peut, comme dans l'exemple précédent, déplacer la tache focale toutes les 1 ms, d'une distance de l'ordre du diamètre rₒ à 6 dB. Le recouvrement entre des taches focales voisines est alors de l'ordre de 80 %.

On constitue ainsi une tache focale équivalente dont la forme, la taille et la répartition d'énergie peuvent être adaptées.

Le temps séparant chaque déplacement de la tache focale et la distance dont la tache focale est déplacée peuvent être choisis de telle sorte que la vitesse de déplacement soit supérieure à la vitesse de diffusion thermique dans la cible, de telle sorte que le profil d'intensité de la tache focale équivalente présente la forme souhaitée. Dans ce cas, les pertes par diffusion au cours du balayage de la zone à traiter sont négligeables.

La figure 5 montre l'évolution de la température au cours du temps, au niveau d'une tache focale équivalente circulaire, après la fin d'un tir.

Les figures 5a à 5c donnent en ordonnée la température, et en abscisse la distance à l'axe de la coupelle, le long d'une droite orthogonale à cet axe. La figure 5a donne la température juste après la fin du tir. La courbe de la figure 5a présente une allure analogue à celle de la figure 4b, l'augmentation de température étant sensiblement proportionnelle à l'intensité rayonnée. Les figures 5b et 5c donnent la température 2 s et 4 s après la fin du tir. Les figures 5b et 5c mettent en évidence les effets de la diffusion thermique sur la température. La température aux bords de la tache focale équivalente descend progressivement, tandis que la température au centre reste sensiblement constante.

L'invention propose, afin de limiter la quantité d'énergie rayonnée, et pour maintenir une température constante sur l'ensemble de la tache focale équivalente, de modifier la répartition d'intensité sur la tache focale équivalente dans les tirs suivants. Comme le montre la figure 5, la température descend sur le périmètre de la tache focale équivalente, mais reste à peu près constante au centre de celle-ci. L'invention propose donc de commencer dans un premier tir par un balayage complet de la tache focale équivalente, puis dans les tirs suivants d'entretenir la température sur l'ensemble de la tache focale équivalente en n'irradiant que la zone du périmètre de celle-ci.

La figure 6 montre une répartition d'intensité sur la tache focale équivalente, dans des tirs successifs, permettant de maintenir une température constante. La figure 6a montre le profil d'intensité rayonnée après un premier tir de 0.1 s. La courbe de la figure 6a a la même allure que la courbe de la figure 4b, et correspond à un balayage homogène de la tache focale équivalente. La courbe de la figure 6b montre le profil d'intensité rayonnée dans un tir situé entre 2 s et 3 s après le premier tir et correspond à un balayage de la zone du périmètre de la tache focale équivalente. Le profil d'intensité de la figure 6b permet de compenser les pertes par diffusion, représentées à la figure 5b. De la même façon, la courbe de la figure 6c montre le profil d'intensité rayonnée dans un tir entre 3 s et 4 s après le premier tir et correspond à un balayage de la zone du périmètre de la tache focale équivalente. Le profil d'intensité de la figure 6c permet de compenser les pertes par diffusion, représentées à la figure 5c.

De la sorte, l'invention permet de limiter l'énergie irradiée vers la zone à traiter, en n'émettant après le premier tir que la quantité d'énergie nécessaire pour compenser la diffusion thermique. On arrive ainsi à maintenir une température sensiblement constante au niveau de la tache focale équivalente, pendant des durées importantes.

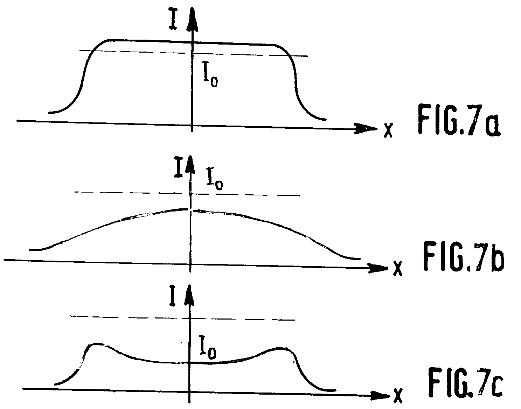

L'invention propose aussi d'utiliser un principe similaire lors de la destruction de cibles en trois dimension. Dans le cas de telles cibles, on traite successivement différentes couches (ou plans) de la cible. La figure 7 montre une répartition d'intensité dans le plan de la tache focale équivalente, et dans un plan situé en avant; la figure 7a montre après un tir la répartition de l'intensité dans le plan de la tache focale équivalente, le long d'une droite traversant la tache focale équivalente. La figure 7a est analogue aux figures 4b ou 6a. La figure 7b montre la répartition d'intensité le long d'une droite parallèle, dans un plan situé en avant, i.e. plus proche du transducteur. On a noté Iₒ, comme sur la figure 4, l'intensité correspondant au seuil de destruction. Comme le montre la figure 7b, lors du tir, une certaine quantité d'énergie est aussi déposée en avant du plan de la tache focale équivalente. L'invention propose de tenir compte de ce fait, lors du traitement dans ce plan.

Autrement dit, dans le cas de cibles en trois dimensions, on découpe la cible en couche, et on commence dans un premier tir par traiter la couche la plus profonde, en choisissant le nombre de déplacements de la tache focale pour couvrir la cible dans la couche en cause, comme indiqué plus haut en référence à la figure 4. Une fois cette couche balayée, de façon sensiblement homogène, on traite la couche suivante, située un peu plus près du transducteur. Pour cette couche, on tient compte que le premier tir a conduit à déposer une certaine quantité d'énergie dans la couche en cause, comme représenté sur la figure 7b. Il peut suffire, pour assurer une destruction des cellules dans la deuxième couche, de compléter cette énergie. La figure 7c montre le long de la même droite que celle de la figure 7b, la répartition d'intensité rayonnée lors du deuxième tir.

De la sorte, on diminue encore la quantité totale d'énergie rayonnée dans la cible, tout en assurant une destruction efficace.

A titre d'exemple, on peut envisager un cible de forme cubique, de 10 mm de côté. On peut traiter cette cible en 5 couches successives. La première couche (la plus profonde) est par exemple traitée lors d'un premier tir, en 0,4 s par balayage uniforme de la surface carrée, à l'aide de 100 déplacements de la tache focale. La deuxième couche, située 2 mm au dessus est traitée immédiatement après, en un deuxième tir, en une durée de 0,2 s, non pas par balayage uniforme du carré, mais comme décrit à la figure 7b, en insonifiant plus spécialement les bords du carré pour compléter l'énergie déposée sur la deuxième couche lors du premier tir. On continue de cette façon, de sorte à traiter l'ensemble de la cible en une durée de l'ordre de 1 s, pendant laquelle la diffusion thermique est négligeable.

Ceci correspond à un mode de traitement dans lequel on souhaite détruire l'ensemble du volume de la cible. Il est aussi possible de ne traiter que la surface d'une cible tridimensionnelle, pour en nécroser le pourtour.

Les modes de réalisation de l'invention décrits en référence aux figures 6 et 7 peuvent être combinés. Il est donc possible de tenir compte en même temps de l'évolution de la température au cours du temps par diffusion thermique et de la présence d'énergie en dehors du plan de la tache focale équivalente.

Il est proposé, selon l'invention de déterminer l'allure de la cible à traiter, puis de simuler par ordinateur les différents tirs du traitement, de façon à prendre en compte la diffusion thermique et/ou la présence d'énergie en dehors du plan de la tache focale équivalente. La forme de la tache focale étant préalablement déterminée, l'ordinateur calcule pour chaque position de la tache focale, la température atteinte à la position suivante de la tache focale du fait de l'énergie déjà rayonnée. L'ordinateur ajuste l'intensité pour la position suivante de la tache focale, de façon à obtenir la température souhaitée. Ceci permet de déterminer pour tous les point de la cible la quantité d'énergie nécessaire et suffisante pour assurer la destruction, en limitant les risques de brulûre dus à une énergie excédentaire.

Une telle simulation par ordinateur peut s'effectuer par itérations successives, pour bien prendre en compte le fait que chaque tir réagit aussi sur la tir précédent. Toutefois, il faut remarquer que si l'intensité a été correctement réglée, les tissus peuvent être détruits lors du premier passage, et dans ces conditions, (comme la transmission des ultrasons s'effectue mal dans les zones brûlées, les zones déjà traitées sont peu affectées par les tirs ultérieurs, ce qui simplifie les calculs. D'autres méthodes de calcul et de simulation peuvent aussi être envisagées.

Bien entendu, il est possible de modifier la répartition d'énergie rayonnée, quelle que soit la forme de la tache focale équivalente, autre que les formes cubiques ou circulaires données à titre d'exemple, de façon à s'adapter aux déperditions par diffusion; on peut ainsi maintenir constante la température sur l'ensemble de la tache focale équivalente ou du volume de la cible, en limitant l'énergie rayonnée. Ceci permet en outre de limiter la montée en température en dehors de la tache focale équivalente.

Pour permettre une variation de l'intensité sur la tache focale équivalente, qui peut être nécessaire pour ce type de traitement, il est possible de faire varier le pas de déplacement entre chaque position de la tache focale. Comme décrit plus haut, un recouvrement optimal de la tache focale équivalente est obtenu lorsque le pas est égal au diamètre à 6 dB de la tache focale.

Il est aussi possible de faire varier la durée du signal d'excitation pour une position de la tache focale; dans l'exemple donné plus haut, cette durée est de 1ms; elle peut par exemple varier dans une plage de 0,1 ms à 20 ms.

La durée minimale est dictée par le fait qu'un train d'ondes doit comporter un nombre d'oscillations suffisant pour qu'un fonctionnement correspondant au régime permanent soit atteint pendant un temps largement supérieur aux régimes transitoires qui apparaissent au début et à la fin du train d'onde, soit:
- stabilisation de l'amplitude et de la forme de l'onde émise, i.e. 4 à 5 oscillations pour une céramique bien adaptée, soit 4 à 5 µs pour 1 MHz;
- au niveau du foyer, large recouvrement des trains d'ondes issus de céramiques différentes, dans le cas le plus défavorable, i.e. 5 µs environ, soit 5 oscillations à 1 MHz.

Dans ce cas, la durée des régimes transitoires est au maximum de l'ordre de 10 µs. La durée minimale de 0,1 ms assure donc un traitement efficace.

Pour ce qui est de la durée maximale d'émission en une position donnée de la tache focale, il faut noter que le volume total à traiter doit être exploré pendant un temps suffisamment bref, pour que la diffusion thermiquene modifie pas sensiblementla répartition des températures; par exemple, en fonction du volume, une durée totale de 1 à 5 secondes peut convenir. Cette durée totale dépend du nombre de points et de la durée d'émission en chaque point.

Ceci permet de déterminer la durée d'émission en chaque point de la tache focale. Pour 1000 points, cette durée peut aller jusqu'à 1 ms; pour 100 points, elle peut atteindre 10 ms. Un choix de 20 ms pour la durée maximale est donc raisonnable.

Enfin, il est possible de faire varier la durée des blancs pendant lesquels la position de la tache focale varie, dans le cas où il existe de tels blancs.

L'invention permet ainsi de modifier l'intensité de l'irradiation, sans pour autant impliquer des variations des amplitudes des signaux d'excitation et des signaux ultrasonores émis par les éléments piézo-électriques. Ceci simplifie considérablement la structure du dispositif utilisé.

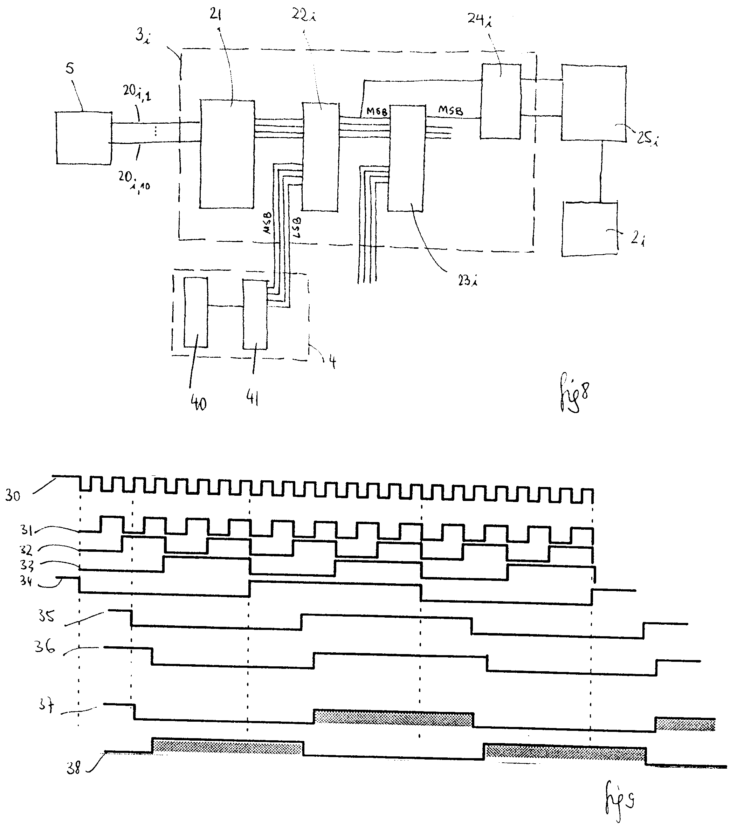

La figure 8 montre un schéma d'un autre mode de réalisation du dispositif de la figure 1.

L'émetteur 3ᵢ de la figure 8 reçoit du générateur d'adresse 5 un signal d'adresse, donnant la position du foyer sur 10 bits, via une pluralité de conducteurs 20_{i,1} à 20_{i,10}.

Il reçoit aussi du pilote haute fréquence 4 un signal pilote . Soit f la fréquence d'émission du signal ultrasonore; on choisit, comme expliqué plus haut, de déphaser les signaux des éléments piézo-électriques d'un multiple d'un seizième de phase. Le pilote haute fréquence 4 comprend une horloge 40 à fréquence 16.f, qui attaque un compteur décimal à quatre bits 41. En sortie du compteur 41, le bit de poids fort (MSB) donne donc un signal en créneaux rectangulaires à la fréquence f.

L'émetteur 3ᵢ comprend des moyens de mémoire, par exemple une mémoire morte type ROM 21ᵢ (ou tout autre type de mémoire) , qui reçoit en entrée les signaux de position du foyer sur les conducteurs 20_{i,1} à 20_{i,10}. En sortie, la mémoire 21ᵢ émet sur quatre bits un signal de déphasage.

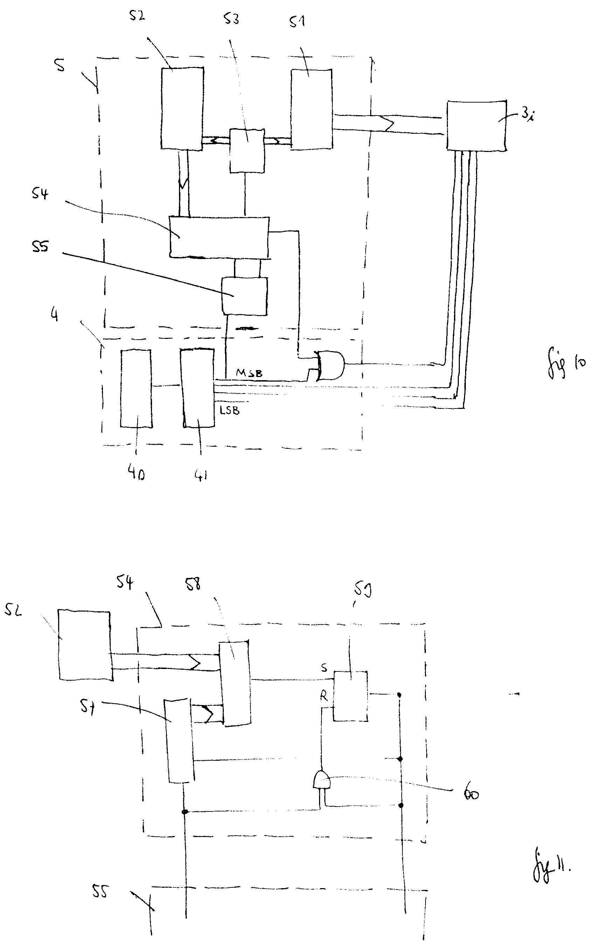

L'émetteur 3ᵢ comprend un additionneur 2x4 bits 22ᵢ qui reçoit sur une entrée les quatre bits émis en sortie par la mémoire ROM 21ᵢ, et sur son autre entrée les quatre bits provenant du compteur 41 du pilote HF 4 (le cas échéant via une porte supplémentaire, comme décrit en référence à la figure 10). De la sorte, on obtient sur le bit de poids fort (MSB) en sortie de l'additionneur 22ᵢ le signal d'horloge à la fréquence f, avec un retard correspondant à la valeur de déphasage donnée par la mémoire ROM 21ᵢ.

L'émetteur 3ᵢ comprend un deuxième additionneur 2x4 bits 23ᵢ, permettant d'introduire un deuxième décalage constant. L'additionneur 23ᵢ reçoit sur une entrée le signal de sortie de l'additionneur 22ᵢ, et sur son autre entrée un signal sur quatre bits, permettant le réglage de la valeur du temps mort entre deux créneaux de commande. Le signal de réglage de la valeur du temps mort est fixé, pour tous les éléments piézo-électriques, en fonction du temps mort devant séparer deux signaux d'attaque des transistors push-pull d'un dispositif d'émission 25ᵢ associé à un élément piézo-électrique 2ᵢ.

L'émetteur 3ᵢ comprend une porte 24ᵢ qui reçoit en entrée le bit de poids fort émis en sortie par l'additionneur 22ᵢ et le bit de poids fort émis en sortie par l'additionneur 23ᵢ. Les sorties ET et NON-OU de la porte 24ᵢ commandent le dispositif d'émission 25ᵢ, en fournissant les signaux d'attaque des transistors push-pull.

La figure 9 montre un chronogramme des signaux dans le circuit de la figure 8. Apparaissent sur la figure 9 le signal 30 à la fréquence 16.f émis par l'horloge 40; les signaux 31 à 34 de sortie du compteur 41, le signal 34 correspond au bit de poids fort à la fréquence f; le signal 35 du bit de poids fort en sortie de l'additionneur 22ᵢ, déphasé par rapport au signal 34 du retard fourni par la mémoire 21ᵢ; le signal 36 du bit de poids fort en sortie de l'additionneur 23ᵢ, déphasé par rapport au signal 35 de la valeur du temps mort; les signaux 37 et 38 de sortie de la porte 24.

Le circuit de la figure 8 est simple et très stable. Sa simplicité permet de le reproduire facilement à un grand nombre d'exemplaires, pour chaque élément piézo-électrique du transducteur.

Le fonctionnement du circuit de la figure 8 est stable, car les valeurs des retards sont appliquées en permanence, et ne sont modifiées que lors des changements de position du foyer; il est donc possible d'appliquer sur les signaux correspondants au retards un filtrage énergique. De même, si un parasite intense vient modifier la valeur, la perturbation ne peut être que transitoire.

La figure 10 montre un mode de réalisation d'un appareil pour la mise en oeuvre de l'invention.

On retrouve sur la figure 10 le pilote haute fréquence 4, avec l'horloge 40 à 16.f et le compteur 41.

Avant le début du traitement, les paramètres du traitement - durée totale du tir, positions successives de la tache focale, et durée d'émission des ultrasons en chaque position de la tache focale - obtenus par exemple par simulation sur ordinateur, sont envoyées à deux mémoires reprogrammables (RAM ou EEPROM) 51 et 52 du générateur d'adresse 5.

Le générateur d'adresses 5 comprend un compteur de points 53, qui contient en permanence le numéro d'ordre de la position courante de la tache focale, par rapport à l'ensemble des positions successives de la tache focale. Le compteur de point 53 fournit en permanence aux mémoires 51 et 52 un signal représentatif de la position courante de la tache focale.

En cours de traitement, la mémoire 51 envoie aux différents émetteurs 3ᵢ les adresses de la position courante de la tache focale, comme expliqué plus haut, via les conducteurs 8_{i,1} à 8_{i,n} (figure 3) ou 20_{i,1} à 20_{i,10} (figure 8).

Le générateur d'adresse 5 comprend en outre un circuit de validation 54, et un circuit de retard 55. Le circuit de validation 54 reçoit un signal numérique fourni par la mémoire 52, et représentatif de la durée d'émission des ultrasons en la position courante de la tache focale.

Le circuit de validation reçoit en outre de l'horloge 40 un signal d'horloge à la fréquence f, à travers le circuit de retard 55 et le compteur 41.

Le circuit de validation, dont la structure est détaillée en référence à la figure 11, contient un compteur et un comparateur. Le circuit de validation compte pendant une durée correspondant au signal de durée fourni par la mémoire 52, et fournit en sortie un signal de validation. Lorsque la valeur du compteur du circuit de validation 54 atteint la valeur cible fournie par la mémoire 52, le circuit de validation 54 cesse de fournir le signal de validation. Le compteur du circuit de validation est alors remis à zéro, et le circuit de retard 55 bloque le signal d'horloge provenant de l'horloge 41. Par ailleurs, la fin du signal de validation est appliquée au compteur de points 53, pour lui indiquer que l'émission des ultrasons est terminée en cette position de la tache focale; le compteur de points adresse alors aux mémoires 51 et 52 un signal représentatif du numéro d'ordre suivant pour la position de la tache focale. Les mémoires 51 et 52 fournissent alors la position correspondante et la durée correspondante.

Après une durée réglable, le circuit de retard 55 laisse de nouveau passer le signal d'horloge vers le circuit de validation 54.

Une porte ET 42 reçoit en entrée le signal de validation fourni par le circuit de validation 54, et le bit de poids fort (MSB) du compteur 40. La sortie de la porte ET 42 est adressée vers les émetteurs 3ᵢ, en tant que signal d'horloge à la fréquence f. De la sorte, les émetteurs 3ᵢ reçoivent un signal dont le bit de poids fort correspond au signal d'horloge, uniquement pendant des durées correspondant à la durée d'émission des ultrasons. Les émetteurs 3ᵢ reçoivent un signal dont les autres bits (bits de poids faible) sont fournis par le compteur 41.

Le circuit de retard 55 permet d'une part de supprimer toute émission au moment de la commutation d'une position de la tache focale à l'autre; d'autre part, il permet de régler, si nécessaire, la puissance moyenne, en augmentant ou diminuant l'intervalle entre les émissions d'ultrasons entre deux positions de la tache focale.

La figure 11 montre un schéma plus précis d'un mode de réalisation du circuit de validation 54 de la figure 10. Le circuit de validation 54 comprend un compteur 57 dont l'entrée est reliée au signal d'horloge provenant du circuit de retard 55. La sortie du compteur 57 est reliée à une entrée d'un comparateur 58 dont l'autre entrée reçoit le signal de durée provenant de la mémoire 52. La sortie du comparateur est reliée à l'entrée S d'une bascule RS 59, qui donne en sortie le signal de validation du circuit de validation 54. De la sorte, la sortie de la bascule 59 est mise à zéro lorsque la valeur fournie par le compteur atteint la durée d'émission des ultrasons, fournie par la mémoire 52.

Le signal de sortie de la bascule 59 est en outre envoyé vers le circuit de retard 55, pour déclencher le retard après une émission. Le signal de sortie est en outre appliqué à une entrée d'une porte 60, dont l'autre entrée reçoit le signal provenant du circuit de retard 55, et dont la sortie est appliquée à l'entrée R de la bascule 59. Ainsi, la porte 60 est débloquée pour permettre la remise à "1" de la bascule. Le signal de sortie de la bascule 59 est en outre appliqué à l'entrée de remise à zéro du compteur 57, de sorte à remettre à zéro le compteur à la fin d'une émission.

Lorsque le circuit de retard 55 cesse de bloquer le signal d'horloge, le premier front du signal d'horloge remet la bascule à l'état "1", et fait redémarrer le compteur.

Les dispositifs des figures 8 à 11 permettent de mettre en oeuvre l'invention, par des circuits simples et stables.

La figure 12 montre une forme possible de tache focale et de tache focale équivalente selon l'invention. La tache focale et la tache focale équivalente de la figure 12 présentent une forme allongée et permettent de limiter encore les effets d'un mouvement de la zone à traiter. La tache focale est représentée en traits pleins sur la figure 12: elle présente une forme allongée, obtenue par exemple dans un appareil tel que celui des figures 2 et 3 en introduisant un déphasage dans certains émetteurs 3ᵢ. Avantageusement, la tache focale présente une forme allongée dans la direction du mouvement de la zone à traiter. De ce fait, le rapport entre la surface balayée et la surface de la tache focale équivalente est plus faible que dans le cas d'une tache focale circulaire, et l'influence des mouvements de la zone à traiter est réduite.

Selon l'invention, on commande l'appareil de traitement de la façon suivante. On détermine, par des moyens connus la position et les dimensions de la cible à traiter. On détermine ensuite le nombre de couches nécessaires pour le traitement, et la forme de la cible dans chaque couche.

En fonction de la nature de la cible, et de la profondeur de traitement on détermine la fréquence des ondes ultrasonores de traitement.

On choisit une taille et le cas échéant une forme de tache focale équivalente, le cas échéant dans chaque couche. On détermine ensuite, pour cette tache focale équivalente, le temps de traitement et la puissance ultrasonore nécessaires pour assurer la destruction des cellules. On procède ensuite comme dans l'art antérieur au traitement. Toutefois, on évite les inconvénients de l'art antérieur, et par exemple ceux liés à un balayage d'une cible mobile. De fait, la tache focale équivalente selon l'invention est créée à une vitesse telle que les déplacements de la cible sont négligeables.

Le temps de traitement, dans un dispositif de l'art antérieur présentant une tache focale d'un diamètre d'environ 2 mm, est typiquement de l'ordre de 0.1 s, pour une fréquence ultrasonore de 1 MHz et une puissance acoustique de l'ordre de 10 kW. Ce temps de 0.1 s est inférieur au temps de stabilisation, de l'ordre de 2 s (temps au bout duquel la diffusion thermique compense les apports d'énergie par les ondes ultrasonores).

Pour une tache focale équivalente de taille plus importante, le temps de stabilisation augmente dans le rapport des diamètres; ainsi, dans le cas de l'exemple ci-dessus, d'une tache focale équivalente d'une surface cinquante fois supérieure à la tache focale, et présentant un diamètre de l'ordre de 15 mm, le temps de stabilisation est de l'ordre de 15 s. Du fait de l'augmentation du rendement énergétique, le temps nécessaire pour détruire les cellules à l'intérieur de la tache focale équivalente n'est pas multiplié par cinquante, mais est de l'ordre de 2.5 s, pour une fréquence et une puissance équivalentes. De la sorte, le temps de traitement selon l'invention est encore inférieur au temps de stabilisation. Ce temps est aussi suffisamment faible pour que l'appareil soit peu sensible aux mouvement de la zone à traiter.

Bien entendu, la présente invention n'est pas limitée aux modes de réalisation décrits et représentés mais elle est susceptible de nombreuses variantes accessibles à l'homme de l'art sans que l'on ne s'écarte de l'esprit de l'invention. Ainsi, le balayage de la tache focale équivalente par la tache focale peut se faire de multiples façons. Les déplacements de la tache focale peuvent se faire dans un plan, mais aussi dans l'espace. La tache focale équivalente peut avoir la forme d'une sphère, ou encore d'un ellipsoïde, ou plus généralement toute forme en deux ou trois dimensions.

Enfin, il est clair que l'on peut, pour déplacer la tache focale, utiliser des lignes à retard ou tout autre mécanisme équivalent à la place de dispositifs de déphasage. On entend donc par "variation des phases" couvrir aussi le cas de retards équivalents à des variations de phases. On peut aussi, notamment dans le cas de sondes de faibles dimensions, faire varier la position de la tache focale par rapport au transducteur en utilisant des dispositifs mécaniques, tels que des miroirs mobiles ou des prismes mobiles. On peut aussi choisir de déplacer mécaniquement l'ensemble du transducteur, et dans ce cas la tache focale se déplace par rapport au support du transducteur, qui est généralement fixe par rapport au patient à traiter. Quelque soit le système choisi, il est clair que l'invention implique un déplacement de la tache focale par rapport à la zone à traiter, qui peut être atteint par différents moyens.

## Revendications

**1.-** Procédé de commande d'un appareil de traitement par hyperthermie à l'aide d'ultrasons comprenant un transducteur (1) avec une pluralité d'éléments piézo-électriques (2ᵢ) excités chacun par un signal électrique, ledit procédé consistant à émettre des ultrasons focalisés sur une tache focale, et à faire varier la position de ladite tache focale par rapport audit transducteur ou par rapport au support du dit transducteur, la position de la tache focale variant par rapport au transducteur ou par rapport au support du dit transducteur à une vitesse supérieure à la vitesse de diffusion thermique dans la zone à traiter, caractérisé en ce que la puissance de crête de la tache focale et le recouvrement entre les différentes positions de la tache focale sont tels que l'énergie rayonnée au cours d'un tir en tout point de la tache focale équivalente ainsi constituée est sensiblement égale à l'énergie nécessaire pour détruire les cellules.

**2.-** Procédé selon la revendication 1, caractérisé en ce que la position de ladite tache focale par rapport audit transducteur varie grâce aux seules variations des phases relatives des signaux électriques excitant lesdits éléments piézo-électriques

**3.-** Procédé selon la revendication 1 ou 2, caractérisé en ce que la dite tache focale équivalente est balayée au cours d'un tir de façon homogène.

**4.-** Procédé selon la revendication 1 ou 2, caractérisé en ce que ladite tache focale équivalente est balayée au cours d'un tir de façon non homogène de sorte à compléter l'énergie provenant du ou des tirs précédents.

**5.-** Procédé selon l'une des revendications 1 à 4, caractérisé en ce que les ultrasons sont émis au cours d'un tir sous forme de trains d'ondes séparés par des blancs, et en ce que la position de ladite tache focale varie par rapport au transducteur pendant lesdits blancs.

**6.-** Procédé selon l'une des revendications 1 à 4, caractérisé en ce que les ultrasons sont émis au cours d'un tir sous forme de trains d'ondes continus, et en ce que la position de ladite tache focale varie par rapport au transducteur sans interruption de l'émission des ultrasons.

**7.-** Procédé selon l'une quelconques des revendications 1 à 6, caractérisé en ce que ladite tache focale présente une forme allongée, et en ce qu'elle se déplace par rapport au transducteur sensiblement selon sa direction longitudinale.

**8.-** Appareil de traitement par hyperthermie, comprenant un transducteur (1) avec une pluralité d'éléments piézo-électriques (2ᵢ) excités par une pluralité de signaux électriques d'excitation, et des moyens de déphasage des dits signaux électriques d'excitation,
caractérisé en ce qu'il comprend une pluralité d'émetteurs (3ᵢ) émettant chacun un signal électrique d'excitation vers un des dits éléments piézo-électriques (2ᵢ), un pilote haute fréquence (4) émettant un signal pilote commun (7ᵢ) vers les dits émetteurs (3ᵢ), et un générateur d'adresses (5), et en ce que les moyens de déphasage comprennent dans chacun des dits émetteurs (3ᵢ) des moyens (9ᵢ, 10ᵢ) pour déphaser le dit signal pilote (7ᵢ) en fonction de l'adresse reçue depuis le dit générateur d'adresses (5).

**9.-** Appareil de traitement par hyperthermie selon la revendication 8, caractérisé en ce que, dans chacun des dits émetteurs (3ᵢ) les moyens pour déphaser le dit signal pilote (7ᵢ) comprennent un registre à décalage (10ᵢ) et des moyens de mémoire (9ᵢ) commandant le dit registre à décalage en fonction de l'adresse reçue depuis le dit générateur d'adresses (5), et en ce que les dits moyens de mémoire (9ᵢ) sont préprogrammés en fonction de la position de l'émetteur (3ᵢ) dans lequel ils se trouvent.

**10.-** Appareil de traitement par hyperthermie selon la revendication 9, caractérisé en ce que chacun des dits registres à décalage (10ᵢ) est susceptible d'induire sur le signal pilote des déphasages variant de seizième de période en seizième de période.

**11.-** Appareil de traitement par hyperthermie selon la revendication 9, caractérisé en ce que chacun des dits registres à décalage (10ᵢ) est susceptible d'induire sur le signal pilote des déphasages variant de huitième de période en huitième de période.
